# EUROPÄISCHE PATENTSCHRIFT

(11) **EP 3 067 008 B1**
(45) Veröffentlichungstag und Bekanntmachung des Hinweises auf die Patenterteilung: **02.08.2017**
(21) Anmeldenummer: 16159437.9
(22) Anmeldetag: 09.03.2016
(51) Int. Cl.: A61C 1/12, A61B 17/16, A61C 1/06

(54) **DENTALES ODER CHIRURGISCHES HANDSTÜCK**
DENTAL OR SURGICAL HANDPIECE
PIÈCE À MAIN CHIRURGICALE OU DENTAIRE

(30) Priorität: 09.03.2015 DE 102015103432; 20.07.2015 DE 102015111757
(43) Veröffentlichungstag der Anmeldung: 14.09.2016
(73) Patentinhaber: SycoTec GmbH & Co. KG, 88299 Leutkirch im Allgäu (DE)
(72) Erfinder: Dr. Rickert, Martin, 88410 Bad Wurzach-Haidgau (DE); Rickert, Carolin, 88410 Bad Wurzach-Haidgau (DE); Mack, Karl, 87452 Altusried (DE)
(74) Vertreter: Pfister & Pfister Patent- und Rechtsanwälte

(56) Entgegenhaltungen:
- EP-A1- 2 514 386
- EP-A1- 2 581 061

## Beschreibung

Die Erfindung betrifft ein dentales oder chirurgisches Handstück. Dieses weist einen Kopf auf, der dazu dient, ein Werkzeug aufzunehmen. Es weist auch eine Anschlusshülse auf, die für die Kopplung des Handstücks an einen Antriebsmotor bestimmt ist. Der Kopf und die Anschlusshülse sind durch ein Verbindungsstück verbunden. Das Verbindungsstück lagert einen Antriebsstrang, der dazu dient, eine Rotationsenergie von der Anschlusshülse in den Kopf für den Antrieb des Werkzeuges zu übertragen.

Derartige Handstücke werden typischerweise verwendet, um Werkzeuge für diverse Behandlungen am menschlichen oder tierischen Körper vorzunehmen. Beispielsweise kann es sich bei einem solchen Werkzeug um einen Bohrer für Zähne oder um ein Schneidewerkzeug für chirurgische Eingriffe handeln. Bekannte Handstücke sind typischerweise aus Metall, insbesondere mit einer metallenen Außenhaut gefertigt. Beispielhaft sei in diesem Zusammenhang die EP2581061 A1 genannt. Im medizinischen Bereich herrscht grundsätzlich eine besonders hohe Anforderung an die Hygiene und die Keimfreiheit von Instrumenten. Deshalb ist es typischerweise erforderlich, die verwendeten Werkzeuge und Handstücke regelmäßig, beispielsweise nach jeder Benutzung oder am Ende eines Tages, zu säubern und zu desinfizieren oder zu sterilisieren. Dies kann beispielsweise in einem Autoklaven erfolgen.

Im Fall einer behördlichen Überprüfung oder auch im Fall einer beim Patienten auftretenden Infektion kann es erforderlich sein, die korrekte Säuberung und Sterilisation eines Werkzeugs oder eines Handstücks nachzuweisen. Hierzu werden derartige Vorgänge regelmäßig dokumentiert, was typischerweise manuell erfolgt und einen erheblichen Aufwand erfordert.

Es ist deshalb eine Aufgabe der Erfindung, ein Handstück vorzusehen, welches sich beispielsweise hinsichtlich der Dokumentationen einfacher handhaben lässt.

Dies wird erfindungsgemäß durch ein Handstück nach Anspruch 1 erreicht. Vorteilhafte Ausgestaltungen können beispielsweise den Unteransprüchen entnommen werden.

Die Erfindung betrifft ein dentales oder chirurgisches Handstück. Dieses weist einen Kopf auf, der dazu dient, ein Werkzeug aufzunehmen. Es weist auch eine Anschlusshülse auf, die für die Kopplung des Handstücks an einen Antriebsmotor bestimmt ist. Der Kopf und die Anschlusshülse sind durch ein Verbindungsstück verbunden. Das Verbindungsstück lagert einen Antriebsstrang, der dazu dient, eine Rotationsenergie von der Anschlusshülse in den Kopf für den Antrieb des Werkzeuges zu übertragen.

Erfindungsgemäß ist vorgesehen, dass das Handstück einen RFID-Transponder und einen das Handstück begrenzenden Kunststoffmantel aufweist, und der RFID-Transponder von dem Kunststoffmantel geschützt ist.

RFID bezeichnet eine Technologie für Sender-Empfänger-Systeme zum automatischen und berührungslosen Identifizieren und Lokalisieren von Objekten mit Radiowellen.

Ein RFID-System besteht aus einem RFID-Transponder (auch Funketikett genannt), der sich am oder im Gegenstand befindet und einen kennzeichnenden Code enthält. Der RFID-Transponder wirkt mit einem Lesegerät zusammen.

Die Kopplung geschieht durch vom Lesegerät erzeugte, hochfrequente Radiowellen. Damit werden nicht nur Daten übertragen, sondern auch der Transponder mit Energie versorgt. Die Datenübertragung erfolgt dabei zum Beispiel unidirektional, vom Transponder zum Lesegerät (unter Verwendung der übermittelten Energie) oder auch bidirektional, wo auf den Transponder auch geschrieben wird.

Mittels des RFID-Transponders ist eine automatisierte Erkennung des Handstücks möglich. Damit kann beispielsweise bei Reinigungs- oder Sterilisationsvorgängen das jeweils durchführende Gerät, beispielsweise ein Autoklav, automatisiert das jeweilige Handstück erkennen. Hierzu können in dem RFID-Transponder Daten wie beispielsweise der Typ, der Hersteller oder eine Seriennummer des Handstücks gespeichert sein. Dies ermöglicht es, Dokumentationen zu automatisieren.

Beispielsweise ist es in einer einfachen Ausführung möglich, einen lediglich auslesbaren RFID-Transponder zu verwenden, welcher die oben genannten oder auch weitere Daten enthalten kann. In diesem Fall kann der RFID-Transponder beispielsweise herstellerseitig mit den entsprechenden Daten programmiert werden. Ein Autoklav oder ein anderes zur Reinigung, Sterilisierung oder Desinfektion verwendetes Gerät kann beispielsweise die Daten auslesen und insbesondere zusammen mit weiteren gegebenenfalls erforderlichen Daten wie einem Datum, einer Zeit, einem Bediener oder einer verwendeten Substanz in einer Datenbank abspeichern. Damit kann im Zweifelsfall, beispielsweise bei einer Kontrolle oder im Fall einer Infektion eines Patienten, der Reinigungs- oder Sterilisationsvorgang nachvollzogen und bewiesen werden.

In einer alternativen Ausführung ist es beispielsweise auch möglich, einen RFID-Transponder zu verwenden, welcher auch beschreibbar ist. Damit können nicht nur feststehende Daten, beispielsweise die weiter oben erwähnten Daten, zum Auslesen auf dem RFID-Transponder gespeichert werden, sondern es können auch dynamisch Daten auf den Transponder geschrieben werden. Dies ermöglicht es, die weiter oben bereits erwähnte Dokumentation auf dem Transponder des Handstücks selbst vorzunehmen. Dies kann beispielsweise zusätzlich zu einer Dokumentation in einer Datenbank oder auch alternativ zur Dokumentation in einer Datenbank erfolgen.

Der Kopf kann beispielsweise Aufnahmemittel für das Werkzeug aufweisen, welche auch zum Antrieb des Werkzeugs ausgebildet sein können. Der Antriebsmotor ist typischerweise extern zum Handstück ausgebildet und kann über Mittel wie beispielsweise eine drehbare, typischerweise flexible Welle oder auch mittels Luftdruck auf das Handstück wirken. Typischerweise vermittelt der Antriebsmotor dabei eine Drehbewegung, welche dann wiederum an das Werkzeug weitergegeben wird. Innerhalb des Handstücks erfolgt diese Weitergabe typischerweise mittels des Antriebsstrangs. Unter der Übertragung von Rotationsenergie kann insbesondere die Übertragung einer Rotation verstanden werden.

Das Handstück kann insbesondere autoklavierbar ausgebildet sein oder anderweitig dafür ausgebildet sein, maschinell gereinigt, desinfiziert oder sterilisiert zu werden. Beispielsweise kann dafür eine entsprechende Widerstandsfähigkeit gegen bestimmte Chemikalien oder gegen Wärme vorgesehen sein.

Der Kunststoffmantel ermöglicht in vorteilhafter Weise, dass auf den RFID-Transponder zugegriffen werden kann, obwohl er innerhalb des Handstücks angeordnet ist. Im Rahmen der Erfindung wurde erkannt, dass bekannte Ausführungen von Handstücken, welche mit metallischem Material ummantelt sind, der Verwendung eines RFID-Transponders insofern entgegenstehen, als diese die Ausbreitung der für das Auslesen des RFID-Transponders notwendigen Funkwellen behindern. Dem könnte zwar dadurch abgeholfen werden, dass der RFID-Transponder außenliegend am Handstück angebracht wird, es hat sich jedoch herausgestellt, dass in diesem Fall die zum Reinigen und Desinfizieren verwendeten Substanzen typischerweise verbaute RFID-Transponder schädigen und damit deren Funktionsfähigkeit beeinträchtigen. Die erfindungsgemäße Ausführung eines Handstücks bietet somit einen besonderen Vorteil insofern, als der RFID-Transponder durch den Kunststoffmantel, durch welchen sich Funkwellen problemlos ausbreiten können, leicht auslesbar ist und gleichzeitig gegen Beschädigungen geschützt wird.

Geschickter Weise ist daher auch vorgesehen, dass sich sich zwischen dem RFID-Transponder und der Handauflagefläche, insbesondere in axialer Richtung der Längserstreckung des Handstückes, kein die Funkwellen weiter dämpfendes oder reflektieres Element befindet.

Aus diesem Grund ist vorteilhafterweise vorgesehen, dass um den Kunststoffmantel oder innerhalb des Kunststoffmantels kein weiterer Mantel aus metallischem Material vorgesehen ist, insbesondere nicht außerhalb des RFID-Transponders. Dies kann auch so ausgedrückt werden, dass der Kunststoffmantel die einzige Ummantelung oder Begrenzung des Handstücks ist. Dies gilt insbesondere im Bereich des RFID-Transponders. Insbesondere sollte vorteilhaft ein Bereich von der Außenseite des Handstücks bis zum RFID-Transponder vorgesehen sein, in welchem sich keine metallischen Teile befinden.

Gemäß jeweiligen Ausführungen ist der RFID-Transponder am Kopf, am Verbindungsstück oder an der Anschlusshülse vorgesehen. Damit kann ein vorteilhafter Zugang zum Auslesen unter Berücksichtigung der geometrischen, baulichen und materialmäßigen Gegebenheiten sichergestellt werden.

Das Verbindungsstück ist gemäß einer bevorzugten Ausführung knieartig, abgewickelt ausgebildet ist weist ein dem Kopf zugewandtes Kopfstück und ein der Anschlusshülse zugewandtes Anschlussstück auf. Das Kopfstück oder das Anschlussstück tragen dabei den RFID-Transponder. Dies entspricht einer Ausführung, welche sich in der Praxis als ergonomisch erwiesen hat.

Bevorzugt ist der Kunststoffmantel schalenartig ausgebildet. Damit kann eine vorteilhafte Umschließung des Inneren des Handstücks erreicht werden. Der Kunststoffmantel kann dabei insbesondere mehrteilig ausgebildet sein, beispielsweise zweiteilig, so dass zwei oder mehr Schalen ineinander greifen und/oder sich gegenseitig überlappen. Damit kann der Herstellungsvorgang vereinfacht werden und es kann die Stabilität erhöht werden.

Der Kunststoffmantel kann bevorzugt als Hülse bzw. hülsenartig ausgebildet sein. Auch dies ermöglicht eine vorteilhafte Umschließung.

In einer bevorzugten Ausgestaltung ist dabei vorgesehen, dass der Kunststoffmantel bildet an seiner Oberfläche auch eine Handauflagefläche aus. Der Kunststoffmantel hat mehrere Aufgaben, er schützt effektiv den RFID Transponder und bildet gleichzeitig eine Handauflagefläche, die entsprechend gestaltbar und auch optimierbar ist, um dem Benutzer ein angenehmes Griffgefühl zu geben. Darüberhinaus wird keine zusätzliche Außenhülle aus Metall oder ähnlichem benötigt, was nur Kosten und Gewicht erhöht.

Gemäß einer Ausführung ist der Kunststoffmantel an dem Handstück als tragendes Element eingesetzt oder ausgebildet. Er kann dabei insbesondere zur Abstützung und/oder Lagerung des Antriebsstranges dienen.

Gemäß einer weiteren Ausführung ist vorgesehen, dass der Antriebsstrang als tragendes Element eingesetzt oder ausgebildet ist. Anders ausgedrückt kann dies bedeuten, dass der Antriebsstrang die Form vorgibt und der Kunststoffmantel sich dieser Form im Wesentlichen anpasst.

Der Antriebsstrang kann insbesondere eine jeweilige Anzahl von Stangen, Getrieben, Lagern, Zahnrädern und/oder Kegelrädern aufweisen. Derartige Elemente werden typischerweise mit Strukturen versehen, welche die Position dieser Elemente relativ zueinander fixieren.

Mit Lagern können beispielsweise Stangen gelagert und in ihrer Position gehalten werden, welche eine Drehbewegung übertragen. Die

Lager erlauben dabei typischerweise lediglich eine Drehbewegung, während sie translatorische Bewegungen verhindern. Beispielsweise kann es sich dabei um Kugellager handeln, welche eine besonders niedrige Reibung und einen entsprechend niedrigen Verschleiß aufweisen.

Ein Kegelgelenk kann beispielsweise eingesetzt werden, um eine Drehbewegung auf einen abgewinkelten Teil des Handstücks zu übertragen. Hierfür ist es typischerweise erforderlich, dass üblicherweise zwei Kegelräder des Kegelgelenks exakt zueinander in Position gehalten werden, so dass jeweilige Zähne ineinander eingreifen.

Somit ist typischerweise für das Funktionieren eines Antriebsstrangs eine spezifische Anordnung seiner Elemente zueinander erforderlich. Diese Anordnung soll typischerweise auch während des Betriebs konstant gehalten werden.

Für die Anordnung der Elemente des Antriebsstrangs kann beispielsweise ein Skelett vorhanden sein, welches die Elemente zueinander fixiert. Dieses Skelett ist typischerweise aus starrem Material, beispielsweise Metall, ausgebildet. Insbesondere ist vorteilhaft der RFID-Transponder außerhalb des Skeletts angeordnet, um eine Beeinträchtigung der Kommunikation des RFID-Transponders zu vermeiden.

Gemäß einer weiteren Ausführung ist vorgesehen, dass eine den Antriebsstrang umgebende Hülse als tragendes Element eingesetzt oder ausgebildet ist. Anders ausgedrückt kann dies bedeuten, dass die Hülse die Form vorgibt und der Kunststoffmantel sich dieser Form im Wesentlichen anpasst.

Die Hülse kann insbesondere eine hohle Hülse sein. Sie kann insbesondere aus Metall ausgebildet sein. Der RFID-Transponder ist typischerweise außerhalb der Hülse angeordnet, um eine Beeinträchtigung der Kommunikation des RFID-Transponders zu vermeiden.

Die Hülse kann den Antriebsstrang ganz oder teilweise, typischerweise zumindest überwiegend, umschließen. Insbesondere gilt dies hinsichtlich der weiter oben erwähnten Komponenten des Antriebsstrangs. Damit kann der Antriebsstrang vor mechanischer oder sonstiger Beschädigung geschützt werden.

Mittels der Hülse ist es auch möglich, einfache Fertigungsverfahren zum Aufbringen des Kunststoffmantels zu verwenden. Beispielsweise kann der Kunststoffmantel aufgespritzt werden. Ein Eindringen des Materials des Kunststoffmantels in den Antriebsstrang, was zu einer Störung der Funktion führen könnte, wird durch die Hülse vorteilhaft verhindert.

Insbesondere können an der Hülse Lager für die sich drehenden Elemente des Antriebsstrangs befestigt werden. Beispielsweise können in der Hülse eine Anzahl von Kugellagern befestigt sein, durch welche Stangen drehbar, aber lateral fixiert, gehalten werden, welche eine Drehbewegung übertragen.

Der Kunststoffmantel kann in den eben beschriebenen Fällen, in welchen der Antriebsstrang oder eine Hülse als tragendes Element eingesetzt wird oder ausgebildet ist, so ausgeführt werden, dass er sich der vorgegebenen Form anpasst. Auf eine besondere Festigkeit des Kunststoffmantels kann verzichtet werden. Dies erlaubt beispielsweise die Ausbildung des Kunststoffmantels vollständig oder teilweise aus einem weichen Material, welches dem Benutzer eine angenehme Haptik bietet.

Gemäß einer Ausführung ist der Kunststoffmantel als aufgespritzte Hülle ausgebildet. Gemäß einer weiteren Ausführung ist der Kunststoffmantel als aufgezogener Schlauch ausgebildet. Derartige Ausführungen ermöglichen geeignete Herstellungsverfahren und vorteilhafte Eigenschaften des Handstücks.

Der Kunststoffmantel kann gemäß jeweiligen Ausführungen durchsichtig, transparent oder farblich ausgestaltet sein. Dies ermöglicht die Erkennung des Handstücks und die Unterscheidung von anderen Handstücken, was die Verwendung eines falschen Handstücks in kritischen Situationen wie beispielsweise einer Zahnbehandlung oder einer Operation vermeidet. Außerdem kann durch eine transparente oder durchsichte Ausführung eine Überprüfung von im Handstück befindlichen Einheiten erfolgen, ohne das Handstück dafür auseinander nehmen zu müssen.

Bevorzugt ist der RFID-Transponder mindestens mehrseitig, besonders bevorzugt allseitig in den Kunststoffmantel eingebettet. Dies erlaubt eine sichere Lagerung des RFID-Transponders und einen Schutz gegen Beschädigung oder Verschmutzung.

Gemäß einer bevorzugten Ausführung ist der RFID-Transponder auf oder an einer die Funkwellen reflektierenden Metalloberfläche des Handstückes angeordnet. Damit kann das Auslesen erleichtert werden.

Gemäß einer bevorzugten Ausführung ist der RFID-Transponder auf der, einen stumpfen Winkel aufweisenden Seite des knieartig abgewinkelten Verbindungsstückes angeordnet. Unter der einen stumpfen Winkel aufweisenden Seite wird dabei insbesondere die Seite beziehungsweise der Teil verstanden, welcher im Vergleich zum Anschlussstück abgewinkelt ist und/oder mit dem Anschlussstück einen stumpfen Winkel einnimmt. Dies ermöglicht das Auslesen auf kurze Distanz in typischen Reinigungsgeräten wie Autoklaven, wenn der Kopf oben angeordnet ist und sich auf einer dem RFID-Transponder entsprechender Höhe im Gerät ein Auslesemittel, beispielsweise in Form einer hierzu geeigneten Antenne, befindet.

Bevorzugt weist das Handstück ein Medienrohr und/oder einen Lichtleiter auf. Weiter bevorzugt sind das Medienrohr und/oder der Lichtleiter auf der einen überstumpfen Winkel bildenden Seite des knieartig abgewickelten Verbindungsstückes angeordnet. Unter der einen überstumpfen Winkel bildenden Seite wird dabei insbesondere die Seite beziehungsweise der Teil verstanden, welcher im Vergleich zum Kopfstück abgewinkelt ist und/oder mit dem Kopfstück einen überstumpfen Winkel einnimmt. Mittels eines Medienrohrs können beispielsweise Substanzen wie Wasser zum Spülen oder Desinfektionsmittel an das Werkzeug geliefert werden. Mittels des Lichtleiters ist eine Beleuchtung möglich, welche beispielsweise den mittels des Werkzeugs zu bearbeitenden Bereich eines menschlichen oder tierischen Körpers beleuchten kann. Hierzu kann der Lichtleiter an geeigneter Stelle nach außen geführt sein, so dass das Licht nach außen austreten kann.

Der Kunststoffmantel ist bevorzugt aus Verbundmaterial vom Typ PEEK (Polyetheretherketon) oder Silikon gebildet. Diese Materialien haben sich für die Praxis als vorteilhaft erwiesen.

Polyetheretherketon (abgekürzt PEEK) ist ein hochtemperaturbeständiger thermoplastischer Kunststoff und gehört zur Stoffgruppe der Polyaryletherketone. Seine Schmelztemperatur beträgt 335°C. Silikone sind auch in Temperaturbereichen über 200°C temperaturbeständig, und eignen sich ebenso für einen thermischen Sterilisationsprozess. Im Übrigen umfasst die Erfindung auch alle anderen thermoplastischer Kunststoffe, die eine Schmelztemperatur über 130°C, bevorzugt über 150°C, insbesondere bevorzugt über 180°C aufweist.

Eine äußere Form des Kunststoffmantels ist bevorzugt derart vorgesehen, dass der Greifkomfort des Handstücks optimiert wird. Beispielsweise können hierzu Rillen ausgebildet sein. Es kann auch außenliegend ein weicheres Material verwendet werden. Insbesondere in den weiter oben beschriebenen Fällen, in welchen dem Kunststoffmantel keine tragende oder formgebende Bedeutung zukommt, kann auch der gesamte Kunststoffmantel aus weichem Material ausgebildet sein.

Des Weiteren umfasst die Erfindung auch die Verwendung eines RFID-Transponders für den Zweck der automatisierten Erkennung eines Handstücks, insbesondere wenn dieses Handstück einen Kunststoffmantel aufweist.

In der Zeichnung ist die Erfindung insbesondere in einem Ausführungsbeispiel schematisch dargestellt. Es zeigen:
- Fig. 1a: eine Draufsicht auf ein erfindungsgemäßes Handstück gemäß einem ersten Ausführungsbeispiel,
- Fig. 1b: eine Seitenansicht des Handstücks von Fig. 1a,
- Fig. 2a: eine Draufsicht auf ein erfindungsgemäßes Handstück gemäß einem zweiten Ausführungsbeispiel, und
- Fig. 2b: eine Seitenansicht des Handstücks von Fig. 2a.

In Figuren sind gleiche oder einander entsprechende Elemente jeweils mit den gleichen Bezugszeichen bezeichnet und werden daher, sofern nicht zweckmäßig, nicht erneut beschrieben. Die in der gesamten Beschreibung enthaltenen Offenbarungen sind sinngemäß auf gleiche Teile mit gleichen Bezugszeichen bzw. gleichen Bauteilbezeichnungen übertragbar. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiterhin können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

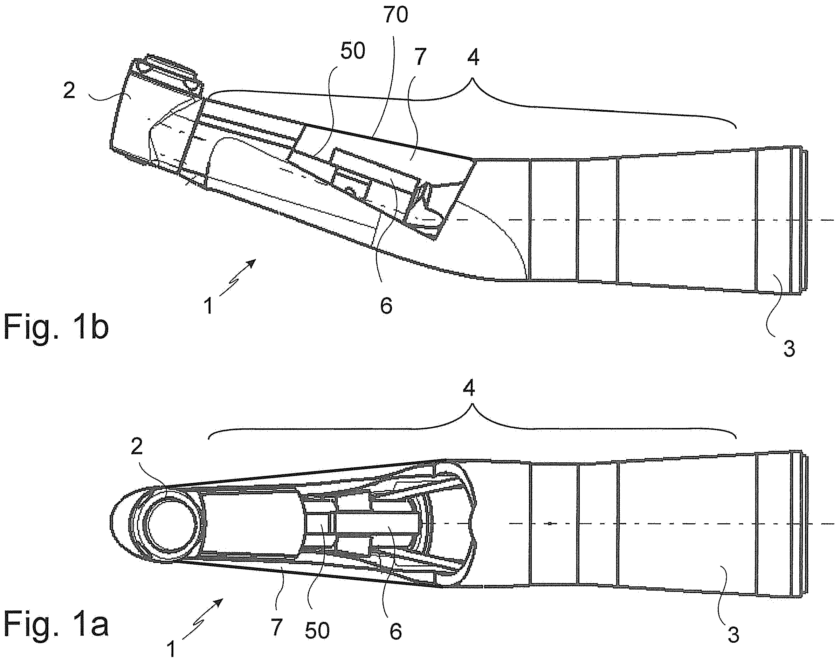

Figur 1 zeigt ein Handstück 1 gemäß einem ersten Ausführungsbeispiel. Dabei zeigt Figur 1a eine Draufsicht und Figur 1b zeigt eine Seitenansicht.

Das Handstück 1 weißt einen Kopf 2, eine Anschlusshülse 3 sowie ein Verbindungsstück 4 auf. Das Verbindungsstück 4 verbindet den Kopf 2 mit der Anschlusshülse 3. Das Verbindungsstück 4 ist wie gezeigt knieartig und leicht abgewinkelt ausgeführt, wobei derjenige Teil des Verbindungsstücks 4, welcher sich näher am Kopf 2 als an der Anschlusshülse 3 befindet, einen stumpfen Winkel zu demjenigen Teil des Verbindungsstücks 4 einnimmt, welcher sich näher an der Anschlusshülse 3 befindet.

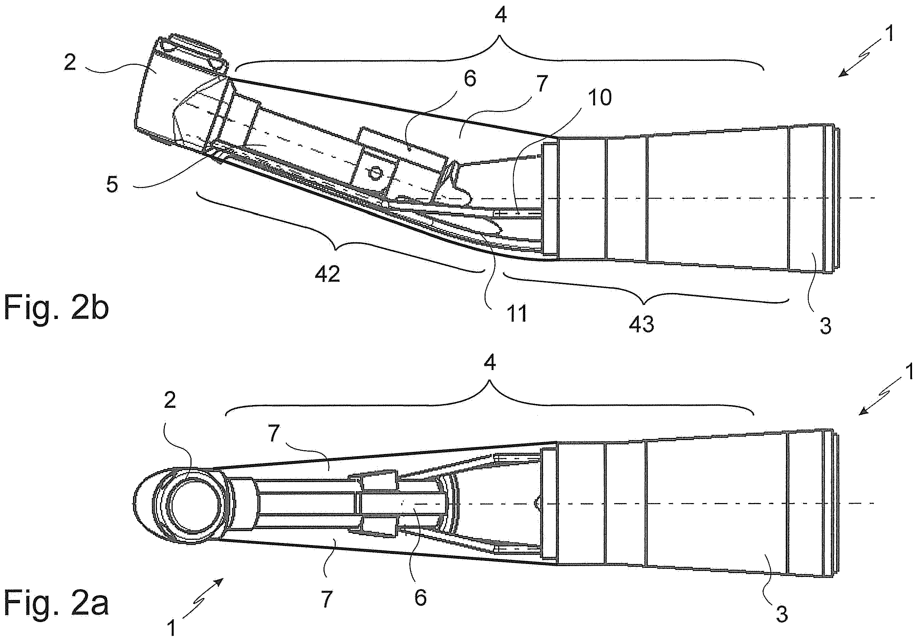

Der Kopf 2 ist dazu ausgebildet, ein nicht dargestelltes Werkzeug aufzunehmen und zu halten. Er ist des Weiteren dazu ausgebildet, eine Drehbewegung auf das Werkzeug auszuüben. Eine solche Drehbewegung wird üblicherweise mittels eines an der Anschlusshülse 3 wirkenden Elektromotors erzeugt und im Verbindungsstück 4 zum Kopf 2 übertragen. Dies wird weiter unten mit Bezug auf Figur 2 noch näher erläutert werden.

Innerhalb des Verbindungsstücks 4 befindet sich eine Metalloberfläche 50, auf welcher ein RFID-Transponder 6 angeordnet ist. Dieser Bereich des Handstücks 1 ist von einem Kunststoffmantel 7 umschlossen. Dieser ist vorliegend als Hülse ausgebildet und ist transparent. Somit ist das Innere des Handstücks 1 sichtbar.

In dem RFID-Transponder 6 sind Daten wie der Hersteller, der Typ, das Herstellungsdatum und die Seriennummer des Handstücks 1 gespeichert. Der RFID-Transponder 6 kann einfach drahtlos mittels eines entsprechenden Auslesegeräts ausgelesen werden, wobei hierzu Funkwellen auf den RFID-Transponder 6 geleitet werden, welche in geeigneter Weise vom RFID-Transponder verändert werden. Dies wird unterstützt durch die Metalloberfläche 50, welche die Funkwellen reflektiert.

Der RFID-Transponder 6 ermöglicht eine besonders einfache, automatisierte Erfassung und eindeutige Identifizierung des Handstücks 1, was beispielsweise die Dokumentation eines Reinigungsvorgangs deutlich erleichtern kann.

Der Kunststoffmantel 7 bildet zumindest abschnittsweise die Außenhaut/Handauflagefläche 70 des Handstücks 1 und bestimmt deren Optik und Haptik. Durch geeignete Wahl des Materials des Kunststoffmantels 7 können somit Handhabbarkeit und Bedienkomfort optimiert werden. Des Weiteren wird das Material vorzugsweise so gewählt, dass typische Reinigungs-, Desinfektions- und Sterilisationsvorgänge unbeschadet überstanden werden. Dies beinhaltet in der Regel Resistenz gegen verwendete Reinigungs- und Desinfektionsflüssigkeiten sowie gegen erhöhte Temperatur beim Sterilisieren. Es kann auch davon gesprochen werden, dass das Handstück 1 und insbesondere der Kunststoffmantel 7 autoklavierbar ausgeführt sind.

Figur 2 zeigt ein zweites Ausführungsbeispiel eines Handstücks 1. Dabei zeigt Figur 2a wiederum eine Draufsicht, wohingegen Figur 2b eine Seitenansicht zeigt.

Das Handstück 1 gemäß dem zweiten Ausführungsbeispiel ist ähnlich ausgebildet wie das Handstück 1 gemäß dem ersten Ausführungsbeispiel. Dabei ist in Figur 2b gezeigt, dass das Verbindungsstück 4 unterteilt ist in ein Kopfstück 42 und ein Anschlussstück 43. Das Kopfstück 42 ist dabei derjenige Abschnitt des Verbindungsstücks 4, welcher näher am Kopf 2 ist als an der Anschlusshülse 3 und welcher auch mit dem Kopf 2 verbunden ist. Das Anschlussstück 43 ist demgegenüber derjenige Abschnitt des Verbindungsstücks 4, welcher näher an der Anschlusshülse 3 ist als am Kopf 2 und welcher auch mit der Anschlusshülse 3 verbunden ist.

Das Kopfstück 42 ist wie gezeigt abgewinkelt im Vergleich zum Anschlussstück 43 ausgeführt. Das Kopfstück 42 nimmt dabei einen stumpfen Winkel zum Anschlussstück 43 ein. Das Kopfstück 42 ist somit diejenige Seite des Verbindungsstücks 4, welche einen stumpfen Winkel aufweist. Umgekehrt bildet das Anschlussstück 43 einen überstumpfen Winkel.

Auch das Handstück 1 gemäß dem zweiten Ausführungsbeispiel weist einen RFID-Transponder 6 auf, welcher an ähnlicher Stelle wie beim ersten Ausführungsbeispiel angeordnet ist. Dieser erfüllt auch den gleichen Zweck, wie er bereits weiter oben mit Bezug auf das erste Ausführungsbeispiel beschrieben wurde.

Im Unterschied zum ersten Ausführungsbeispiel ist jedoch der Bereich des Verbindungsstücks 4, welcher den RFID-Transponder 6 enthält, nicht mit einem aus transparentem Material bestehenden, als Hülse ausgebildeten Kunststoffmantel abgedeckt. Vielmehr ist dieser Bereich mit einem Kunststoffmantel 7 überdeckt, welcher als Spritzmasse ausgebildet ist. Dieser ist grundsätzlich nicht durchsichtig, ist jedoch in den Figuren 2a und 2b durchsichtig dargestellt, so dass die Bestandteile des Handstücks 1 unter dem Kunststoffmantel 7 sichtbar werden.

Der Kunststoffmantel 7 stellt wie auch schon beim ersten Ausführungsbeispiel eine Außenhaut des Handstücks 1 dar und definiert dessen Optik und Haptik. Er umschließt den RFID-Transponder mehrseitig und schützt ihn gegen Einwirkungen von außen.

Unter dem Kunststoffmantel 7 befindet sich des Weiteren ein Antriebsstrang 5, welcher dazu ausgebildet ist, eine an der Anschlusshülse 3 anliegende Rotation auf den Kopf 2 zu übertragen. Im Kopf 2 befindet sich eine in den Figuren nicht sichtbare Halterung für ein nicht dargestelltes Werkzeug, welche dann diese Rotation wiederum auf das Werkzeug übertragen kann. Dies ermöglicht den Antrieb eines in dem Kopf 2 eingespannten Werkzeugs mittels eines nicht dargestellten Motors, welcher an der Anschlusshülse 3 wirkt. Der Antriebsstrang 5 weist vorliegend ein nicht näher dargestelltes Skelett auf, welches einzelne Teile des Antriebsstrangs in einer definierten Positionsbeziehung zueinander hält. Damit wird auch die Form des Handstücks 1 vorgegeben, und zwar einschließlich des Kunststoffmantels 7. Diese Ausführungen gelten entsprechend für das erste Ausführungsbeispiel.

Des Weiteren ist in Figur 2b zu sehen, dass in dem Verbindungsstück 4 ein Medienrohr 10 und ein Lichtleiter 11 verlaufen. Das Medienrohr 10 und der Lichtleiter 11 verlaufen dabei von der Anschlusshülse 3 zu dem Kopf 2.

In dem Medienrohr 10 können Medien wie beispielsweise Wasser zum Spülen oder Chemikalien zur Desinfektion oder zur Beeinflussung von Gewebe, oder auch Medikamente, zu dem Kopf 2 geleitet werden. Von dort aus können Sie an das im Kopf 2 gehaltene Werkzeug weitergegeben werden. Damit kann beispielsweise eine Spülfunktion des Werkzeugs für zahnmedizinische Zwecke betrieben werden.

In dem Lichtleiter 11 kann Licht von der Anschlusshülse 3 zu dem Kopf 2 geleitet werden, so dass mittels einer an der Anschlusshülse 3 angebrachten oder mit dieser zusammenwirkenden, nicht dargestellten Lampe eine Beleuchtung des Kopfs 2 ermöglicht oder einer Umgebung des Kopfs 2 erhellt werden kann. Beispielsweise können geeignete, nicht dargestellte Austrittsfenster für den Lichtleiter 11 vorgesehen sein, so dass das im Lichtleiter 11 geführte Licht an diesen Austrittsfenstern austreten kann. Diese Austrittsfenster können so angeordnet sein, dass ein mit dem Werkzeug, welches im Kopf 2 gehalten wird, zu bearbeitender Bereich beleuchtet wird. Damit kann beispielsweise das Risiko von fehlerhaften Behandlungen verringert werden.

Sowohl beim ersten wie auch beim zweiten Ausführungsbeispiel ist zumindest in dem Bereich des Anschlussstücks 4, in welchem der RFID-Transponder angeordnet ist, kein den RFID-Transponder umgebendes metallisches Bauteil vorgesehen. Insbesondere ist in dem Kunststoffmantel 7 kein Metallanteil vorhanden. Damit wird das Auslesen des RFID-Transponders erheblich erleichtert, da ein Abschirmen von auslesenden Funkwellen durch umgebende Metallteile vermieden wird.

## Patentansprüche

1. Dentales oder chirurgisches Handstück, das einen Kopf (2), der dazu dient, ein Werkzeug aufzunehmen, und eine Anschlusshülse (3), die für die Kopplung des Handstücks (1) an einen Antriebsmotor bestimmt ist, aufweist, und der Kopf (2) und die Anschlusshülse (3) durch ein Verbindungsstück (4) verbunden sind, das Verbindungsstück (4) einen Antriebsstrang (5) lagert, der dazu dient, eine Rotationsenergie von der Anschlusshülse (3) in den Kopf (2) für den Antrieb des Werkzeuges zu übertragen, **dadurch gekennzeichnet, dass** das Handstück (1) einen RFID-Transponder (6) und einen das Handstück (1) begrenzenden Kunststoffmantel (7) aufweist, und der RFID-Transponder (6) von dem Kunststoffmantel (7) geschützt ist.

2. Handstück nach Anspruch 1, **dadurch gekennzeichnet, dass** der RFID-Transponder (6) am Kopf (2), am Verbindungsstück (4) oder an der Anschlusshülse (3) vorgesehen ist.

3. Handstück nach einem der vorhergehenden Ansprüche, **dadurch gekennzeichnet, dass** das Verbindungsstück (4) knieartig, abgewinkelt ausgebildet ist und ein dem Kopf (2) zugewandtes Kopfstück (42) und ein der Anschlusshülse (3) zugewandtes Anschlussstück (43) aufweist, und das Kopfstück (42) oder das Anschlussstück (43) den RFID-Transponder (6) trägt.

4. Handstück nach einem der vorhergehenden Ansprüche, **dadurch gekennzeichnet, dass** der Kunststoffmantel (7) schalenartig ausgebildet ist.

5. Handstück nach einem der vorhergehenden Ansprüche, **dadurch gekennzeichnet, dass** der Kunststoffmantel (7) als Hülse, bzw. hülsenartig oder als aufgezogener Schlauch ausgebildet ist.

6. Handstück nach einem der vorhergehenden Ansprüche, **dadurch gekennzeichnet, dass** der Kunststoffmantel (7) an dem Handstück (1) als tragendes Element eingesetzt ist, insbesondere zur Abstützung bzw Lagerung des Antriebsstranges (5) dient.

7. Handstück nach einem der vorhergehenden Ansprüche, **dadurch gekennzeichnet, dass** der Kunststoffmantel (7) als aufgespritzte Hülle ausgebildet ist.

8. Handstück nach einem der vorhergehenden Ansprüche, **dadurch gekennzeichnet, dass** der Kunststoffmantel (7) bildet an seiner Oberfläche auch eine Handauflagefläche (70) aus.

9. Handstück nach einem der vorhergehenden Ansprüche, **dadurch gekennzeichnet, dass** der Kunststoffmantel (7) durchsichtig, transparent oder farblich ausgestaltet ist.

10. Handstück nach einem der vorhergehenden Ansprüche, **dadurch gekennzeichnet, dass** der RFID-Transponder (6) mindestens mehrseitig, insbesondere allseitig in den Kunststoffmantel (7) eingebettet ist.

11. Handstück nach einem der vorhergehenden Ansprüche, **dadurch gekennzeichnet, dass** der RFID-Transponder (6) auf oder an einer die Funkwellen reflektierenden Metalloberfläche (50) des Handstückes (1) angeordnet ist.

12. Handstück nach einem der vorhergehenden Ansprüche, **dadurch gekennzeichnet, dass** der RFID-Transponder (6) auf der, einen stumpfen Winkel aufweisenden Seite des knieartig abgewinkelten Verbindungsstückes (4) angeordnet ist.

13. Handstück nach einem der vorhergehenden Ansprüche, **dadurch gekennzeichnet, dass** das Handstück (1) ein Medienrohr (10) und/oder einen Lichtleiter (11) aufweist, und das Medienrohr (10) und/oder der Lichtleiter (11) auf der, einen überstumpfen Winkel bildenden Seite des knieartig abgewinkelten Verbindungsstückes (4) angeordnet ist.

14. Handstück nach einem der vorhergehenden Ansprüche, **dadurch gekennzeichnet, dass** der Kunststoffmantel (7) aus Verbundmaterial vom Typ PEEK oder Silikon gebildet ist.

15. Handstück nach einem der vorhergehenden Ansprüche, **dadurch gekennzeichnet, dass** sich zwischen dem RFID-Transponder (6) und der Handauflagefläche (70), insbesondere in axialer Richtung der Längserstreckung des Handstückes, kein die Funkwellen weiter dämpfendes oder reflektieres Element befindet.

## Claims

1. Dental or surgical handpiece comprising a head (2) serving for receiving a tool, and a connector sleeve (3) for coupling the handpiece (1) to a drive motor, and the head (2) and the connector sleeve (3) being connected by a connecting piece (4), the connecting piece (4) accommodating a drivetrain (5) serving for transmitting a rotational energy from the connector sleeve (3) into the head (2) for driving the tool, **characterized in that** the handpiece (1) has an RFID transponder (6) and a plastic casing (7) delimiting the handpiece (1), and the RFID transponder (6) is protected by the plastic casing (7).

2. Handpiece according to claim 1, **characterized in that** the RFID transponder (6) is provided at the head (2), the connecting piece (4), or the connector sleeve (3).

3. Handpiece according to one of the preceding claims, **characterized in that** the connecting piece (4) is bent knee-like and has a head piece (42) facing the head (2), and a connecting piece (43) facing the connector sleeve (3), and the head piece (42) or the connecting piece (43) carries the RFID transponder (6).

4. Handpiece according to one of the preceding claims, **characterized in that** the plastic casing (7) is formed as a shell.

5. Handpiece according to one of the preceding claims, **characterized in that** the plastic casing (7) is formed as a sleeve or sleeve-like or as pulled-on hose.

6. Handpiece according to one of the preceding claims, **characterized in that** the plastic casing (7) is used at the handpiece (1) as supporting element, in particular for supporting or bearing the drivetrain (5).

7. Handpiece according to one of the preceding claims, **characterized in that** the plastic casing (7) is formed as sprayed-on sheath.

8. Handpiece according to one of the preceding claims, **characterized in that** the plastic casing (7) forms a hand rest area (70) on its surface.

9. Handpiece according to one of the preceding claims, **characterized in that** the plastic casing (7) is designed translucent, transparent, or colored.

10. Handpiece according to one of the preceding claims, **characterized in that** the RFID transponder (6) is embedded at least on several sides, in particular on all sides, in the plastic casing (7).

11. Handpiece according to one of the preceding claims, **characterized in that** the RFID transponder (6) is arranged on or at a metallic surface (50) of the handpiece (1) reflecting radio waves.

12. Handpiece according to one of the preceding claims, **characterized in that** the RFID transponder (6) is arranged on the side of the knee-like bent connecting piece (4) which shows an obtuse angle.

13. Handpiece according to one of the preceding claims, **characterized in that** the handpiece (1) has a media tube (10) and/or an optical fiber (11), and the media tube (10) and/or the optical fiber (11) is arranged on the side of the knee-like bent connecting piece (4) forming an obtuse angle.

14. Handpiece according to one of the preceding claims, **characterized in that** the plastic casing (7) is formed from composite material of the PEEK type or silicone.

15. Handpiece according to one of the preceding claims, **characterized in that** between the RFID transponder (6) and the handrest area (70), in particular in axial direction of the longitudinal extension of the handpiece, there is no element further muffling or reflecting the radio waves.

## Revendications

1. Pièce à main chirurgicale ou dentaire possédant une tête (2), servant à recevoir un outil, et une douille de connexion (3) permettant de coupler la pièce à main (1) à un moteur de commande, les deux éléments, la tête (2) et la douille de connexion (3), étant liés entre eux par un élément de liaison (4) de façon à ce que cet élément de liaison (4) comporte un système de transmission (5) destiné à transmettre l'énergie rotationnelle de la douille de connexion (3) vers la tête (2) pour la commande de l'outil, **caractérisée en ce que** la pièce à main (1) comporte un transpondeur RFID (6) et une coque en plastique (7) délimitant la pièce à main (1) et **en ce que** le transpondeur RFID (6) est protégé par la coque en plastique (7).

2. Pièce à main selon la revendication 1, **caractérisée en ce que** le transpondeur RFID (6) est prévu au niveau de la tête (2), au niveau de l'élément de liaison (4) ou au niveau de la douille de connexion (3).

3. Pièce à main selon une des revendications précédentes, **caractérisée en ce que** l'élément de liaison (4) est coudé tel un genou et possède un élément de tête (42) faisant face à la tête (2) et un élément de raccord (43) faisant face à la douille de connexion (3), et **en ce que** l'élément de tête (42) ou l'élément de raccord (43) supporte le transpondeur RFID (6).

4. Pièce à main selon une des revendications précédentes, **caractérisée en ce que** la coque en plastique (7) possède une forme de fourreau.

5. Pièce à main selon une des revendications précédentes, **caractérisée en ce que** la coque en plastique (7) est configurée comme une douille ou comme un manchon gainant.

6. Pièce à main selon une des revendications précédentes, **caractérisée en ce que** la coque en plastique (7) est introduite au niveau de la pièce à main (1) comme élément de support, en particulier destiné au support et au maintien du système de transmission (5).

7. Pièce à main selon une des revendications précédentes, **caractérisée en ce que** la coque en plastique (7) est configurée comme un revêtement issu d'une fabrication par pulvérisation.

8. Pièce à main selon une des revendications précédentes, **caractérisée en ce que** la coque en plastique (7) fournit une surface d'appui-main (70).

9. Pièce à main selon une des revendications précédentes, **caractérisée en ce que** la coque en plastique (7) est translucide, transparente ou colorée.

10. Pièce à main selon une des revendications précédentes, **caractérisée en ce que** le transpondeur RFID (6) est recouvert par la coque en plastique (7) d'au moins de plusieurs côtés et en particulier totalement.

11. Pièce à main selon une des revendications précédentes, **caractérisée en ce que** le transpondeur RFID (6) est situé sur ou devant une surface métallique (50) de la pièce à main (1) réfléchissant les ondes radio.

12. Pièce à main selon une des revendications précédentes, **caractérisée en ce que** le transpondeur RFID est situé au niveau de la partie de l'élément de liaison coudé (4) comportant un angle obtus.

13. Pièce à main selon une des revendications précédentes, **caractérisée en ce que** la pièce à main (1) comporte un tuyau à liquides (10) et/ou un guide de lumière (11) et **en ce que** le tuyau à liquides (10) et/ou le guide de lumière (11) est situé au niveau de la partie de l'élément de liaison coudé (4) comportant un angle obtus.

14. Pièce à main selon une des revendications précédentes, **caractérisée en ce que** la coque en plastique (7) est fabriquée en un matériau composite tel du PEEK ou de la silicone.

15. Pièce à main selon une des revendications précédentes, **caractérisée en ce qu'**aucun élément absorbant ou réfléchissant les ondes radio est situé entre le transpondeur RFID (6) et la surface d'appui-main et en particulier pas selon la direction axiale par rapport à l'orientation en longueur de la pièce à main.
